# EUROPÄISCHE PATENTSCHRIFT

(11) **EP 3 612 789 B1**
(45) Veröffentlichungstag und Bekanntmachung des Hinweises auf die Patenterteilung: **20.12.2023**
(21) Anmeldenummer: 18722914.1
(22) Anmeldetag: 18.04.2018
(51) Int. Cl.: G01B 9/02091, G01B 9/02, A61B 5/00, A61B 3/10

(54) **OCT-BILDERFASSUNGSVORRICHTUNG**
OCT IMAGE CAPTURE DEVICE
DISPOSITIF DE CAPTURE D'IMAGES OCT

(30) Priorität: 18.04.2017 DE 102017108193
(43) Veröffentlichungstag der Anmeldung: 26.02.2020
(73) Patentinhaber: OCUMAX Healthcare GmbH, 30419 Hannover (DE)
(72) Erfinder: LUBATSCHOWSKI, Holger, 30419 Hannover (DE)
(74) Vertreter: Eisenführ Speiser
(86) Internationale Anmeldenummer: PCT/EP2018/059929
(87) Internationale Veröffentlichungsnummer: WO 2018/192988

(56) Entgegenhaltungen:
- WO-A2-2015/189174
- US-A1- 2014 285 812
- US-A1- 2017 035 287
- ANDREI B. VAKHTIN ET AL: "Common-path interferometer for frequency-domain optical coherence tomography", APPLIED OPTICS, Bd. 42, Nr. 34, 1. Dezember 2003 (2003-12-01), Seite 6953, XP055416236, WASHINGTON, DC; US ISSN: 0003-6935, DOI: 10.1364/AO.42.006953

## Beschreibung

Die Erfindung betrifft eine OCT-Untersuchungsvorrichtung zur Erfassung eines Gegenstandes mittels optischer Kohärenztomographie, umfassend eine OCT-Strahlungsquelle, die eine OCT-Strahlung mit einer Wellenlänge von 400nm bis 2000nm und einer spektralen Bandbreite aussendet, die mindestens einen Bereich von 20nm bis 400nm umfasst, oder eine schmale Bandbreite von weniger als 20nm bis 400nm aufweist, wobei die Strahlungsquelle so durchstimmbar ist, dass die schmale Bandbreite eine breitere Bandbreite von 20nm bis 400nm durch zeitversetzte Aussendung von Wellen in unterschiedlichen Wellenlängen ausbildet, einen OCT-Strahlengang, umfassend eine OCT-Ausgangsrichtung der OCT-Strahlung von der OCT-Strahlungsquelle, und eine OCT-Eingangsrichtung einer von einem Bildobjekt rückgestreuten OCT-Strahlung, einen OCT-Strahlungsempfänger für den Empfang der rückgestreuten OCT-Strahlung der OCT-Strahlungsquelle, ein Gehäuse, welches die OCT-Strahlungsquelle und den OCT-Strahlungsempfänger aufnimmt, eine in dem Gehäuse ausgebildete Austrittsöffnung der OCT- Strahlung der OCT-Strahlungsquelle, eine in dem Gehäuse ausgebildete Eintrittsöffnung der rückgestreuten OCT-Strahlung der OCT-Strahlungsquelle, eine OCT-Austrittsrichtung der Strahlung durch die Austrittsöffnung, eine OCT-Eintrittsrichtung der rückgestreuten OCT-Strahlung durch die Eintrittsöffnung, eine Steuerungseinheit, die signaltechnisch mit der OCT-Strahlungsquelle und dem OCT-Strahlungsempfänger verbunden und ausgebildet ist, um in einem Erfassungszeitraum eine Mehrzahl von voneinander beabstandeten Messprofilen zu erfassen und innerhalb des Erfassungszeitraums die OCT-Strahlungsquelle zum Aussenden der OCT-Strahlung und den OCT-Strahlungsempfänger zum Empfang der rückgestreuten OCT-Strahlung anzusteuern.

Die optische Kohärenztomografie (OCT) ist ein Untersuchungsverfahren, bei dem Licht geringer Kohärenzlänge mit Hilfe eines Interferometers zur Entfernungsmessung in streuenden Materialien eingesetzt wird. Die OCT-Untersuchungsmethode wird beispielsweise in der Medizin zur Untersuchung in vivo und in vitro eingesetzt, findet neben dieser medizinischen Anwendung auch in anderen Bereichen zur Untersuchung von streuenden Materialien außerhalb der Medizin Anwendung. Grundsätzlich ist zu verstehen, dass die erfindungsgemäße OCT-Diagnose Vorrichtung und das erfindungsgemäße Verfahren zur OCT-Bilderfassung eines Gegenstands als Diagnostizierverfahren, die am menschlichen oder tierische Körper vorgenommen werden, eingesetzt werden können, jedoch auch als Analyseverfahren in einem anderen Zweck, also unter Ausschluss von solchen Diagnostizierverfahren, die am menschlichen oder tierischen Körper vorgenommen werden, zum Einsatz kommen können. OCT-Untersuchungsmethoden zeichnen sich dadurch aus, dass das untersuchte Material durch den OCT-Untersuchungsvorgang nicht verändert wird und weisen daher weder eine chirurgische noch eine therapeutische Wirkung auf. Es ist zu verstehen, dass das beanspruchte Verfahren zur OCT-Bilderfassung eines Gegenstands in Territorien, die diagnostische Verfahren am menschlichen Körper vom Patentschutz ausschließen, unter Ausschluss des Schutzes solcher Diagnostizierverfahren am menschlichen Körper Gegenstand dieser Beschreibung und der Ansprüche ist.

Bei OCT-Systemen handelt es sich um Messsysteme, die durch Ausrichten eines Messstrahls auf eine idealisiert als Punkt zu betrachtende Fläche des zu untersuchenden Objekts aufgrund der rückgestreuten Strahlung eine punktuelle Messung an diesem Objekt vornehmen. Um die Objektoberfläche und den unmittelbar darunter liegenden Tiefenbereich, bei einer typischen Eindringtiefe von 1 bis 3 mm, über einen größeren Flächenbereich oder insgesamt abzubilden, müssen OCT-Messsysteme daher mittels einer Abrasterung, die typischerweise als Linearbrasterung erfolgt, den Messstrahl über die Oberfläche bewegen. Diese Abrasterung wird bei OCT-Messsystemen durch motorbetriebene Ablenkspiegel erreicht. Eine qualitativ hochwertige OCT-Bilderfassung wird daher so ausgeführt, dass das zu untersuchende Objekt fixiert wird, die OCT-Messvorrichtung in fixierter Position zu dem Objekt angeordnet wird und dann durch eine automatisiert ablaufende Abrasterung mittels des Umlenkspiegels die Oberfläche des Objekts abgerastert wird, um folglich durch eine Vielzahl von Einzelpunktmessungen eine Darstellung der Oberfläche und des unmittelbar unter der Oberfläche liegenden Bereichs zu erhalten.

Diese Messmethode hat sich insgesamt bewährt, erzielt zuverlässige Abbildungsqualitäten und ist mit einer schnellen Messung durchführbar. Nachteilig ist jedoch, dass eine Fixierung des zu untersuchenden Objekts erforderlich ist, um über den Abtastzeitraum eine Relativbewegung zwischen Objekt und der OCT-Messvorrichtung zu vermeiden. Diese Fixierung ist insbesondere bei Einsatz der OCT-Messvorrichtung für diagnostische Zwecke, beispielsweise eine Untersuchung der Netzhaut des menschlichen Auges, nachteilig, da der hierfür notwendige Aufwand zur Fixierung des Kopfes bzw. Auges erheblich ist.

Aus US 2017/0027438 A1 ist ein Operationsmikroskop mit eingekoppelter OCT-Untersuchungsvorrichtung vorbekannt. Der OCT-Messstrahl wird dabei über zwei bewegliche Spiegel und einen feststehenden Spiegel in den Strahlengang des Mikroskops eingekoppelt und folglich dreimalig abgelenkt. Die Leitstrahlengänge des OCT-Strahls sollen durch die zweifach gesteuerte Ablenkung im Wesentlichen parallel zu der optischen Achse des Mikroskops 100 bleiben, um Bilderverzerrungen zu vermeiden. Die insoweit vorbekannte Technologie ist nicht geeignet, um den Winkel zwischen Ausgangsrichtung des OCT-Strahls aus der OCT-Quelle und der Austrittsrichtung des OCT-Strahls aus dem Gehäuse konstant zu halten, sondern kann nur erreichen, dass dieser Winkel im Wesentlichen konstant ist. Eine solche Ausgestaltung, bei der der Winkel jedoch aufgrund der zweifachen Ablenkung und des Prinzips der Abrasterung des Untersuchungsbereichs mittels der zweifachen Ablenkung nicht tatsächlich parallel zur Achse des Mikroskops gehalten werden kann, ist im erfindungsgemäßen Sinne nicht so zu verstehen, dass damit der Winkel zwischen Ausgangsrichtung des OCT-Strahls aus der OCT-Quelle und die Austrittsrichtung des OCT-Strahls aus dem Gehäuse konstant gehalten wird. Die so vorbekannte Vorrichtung erfasst den Untersuchungsbereich mittels der OCT-Vorrichtung durch einen Abrasterungsvorgang und erfordert daher ein Fixieren der Vorrichtung in Bezug auf das zu untersuchende Objekt. Zudem nimmt die Vorrichtung erheblichen Bauraum ein und ist daher unter beengten Raumverhältnissen unhandlich zu benutzen.

Aus US 2014/0285812 A1 ist eine multimodale Abbildungs- und OCT-Messvorrichtung vorbekannt, die in ein Interferometer integriert ist, vorbekannt. Die Daten, die von mehreren Abbildungsmodalitäten parallel erfasst werden, umfassen Messungen von einfach gestreutem, mehrfach gestreutem und diffusem Licht, die eine Charakterisierung verschiedener Tiefenbereiche in der Probe ermöglichen.

Aus WO 2015/189174 A2 ist ein OCT-Messsystem für verbesserte interferometrische Bildgebung vorbekannt, bei dem ein Lichtstrahl in zwei Richtungen über eine Probe gescannt wird und das vom Objekt gestreute Licht mit einem einem räumlich auflösenden Detektor erfasst wird. Der Lichtstrahl kann einen Punkt, eine Linie oder einen zweidimensionalen Bereich auf der Probe beleuchten.

Aus US 2017/035287 A1 ist ein Verfahren zur optimierten Darstellung eines Bildes eines chirurgischen Operationsortes von einem Bildgebungssystem eine interessierende Region an einer ersten Ort innerhalb des Bildes bestimmt wird und ein chirurgisches Daten-Overlays an einer ersten Position erzeugt wird, wobei die erste Position innnerhalb des interessierenden Bereichs zugeordnet wird.

Aus Vakhtin et al: "Common-path interferometer for frequency domain optical coherence tomography", in APPLIED OPTICS, Bd. 42, Nr. 34, 1. Dezember 2003 (2003-12-01), ISSN: 0003-6935 ist ein Michelson-Spektralinterferometer vorbekannt, das einen gemeinsamen Strahlengang für den Referenz- und den Probenstrahlengang verwendet. Diese optische Anordnung wird als kompakter und stabiler als übliche zweiarmige Interferometer beschrieben und soll sich gut für die optische Kohärenztomographie biologischer Proben eignen.

Es besteht daher ein Bedarf für eine OCT-Untersuchungsvorrichtung, die die Durchführung der OCT-Untersuchung insgesamt vereinfacht und gegenüber Messfehlern, die durch eine Relativbewegung zwischen der OCT-Messvorrichtung und dem zu untersuchenden Objekt entstehen, unempfindlicher ist.

Diese Aufgabe wird mit einer OCT-Untersuchungsvorrichtung gemäß der Patentansprüche gelöst, indem die Steuerungseinheit ausgebildet ist, um während des Erfassungszeitraums die OCT-Ausgangsrichtung und die OCT-Austrittsrichtung in ihrer Winkelausrichtung zueinander konstant zu halten.

Erfindungsgemäß wird auch bei der OCT-Untersuchungsvorrichtung nach der Erfindung die Bilderfassung durch Abrastern der Oberfläche des Untersuchungsobjekts mit einem Messstrahl durchgeführt. Diese Bilderfassung erfolgt während eines Erfassungszeitraums, der mit dem Erfassen des ersten Messpunktes in der Abrasterung beginnt und mit dem Erfassen des letzten Messpunktes in der Abrasterung endet. Innerhalb dieses Erfassungszeitraums werden mehrere, also mindestens zwei, in der Regel einige hundert oder tausend Messpunkte mit dem Messstrahl angefahren und hierdurch die Oberfläche beispielsweise zeilenweise oder spiralförmig abgerastert.

Während diese Abrasterung im Stand der Technik durch eine motorisch verstellbaren Umlenkspiegel oder sonstige Strahlablenkvorrichtungen automatisiert erfolgt, wodurch die OCT-Ausgangsrichtung des Strahls aus der OCT-Strahlungsquelle hinsichtlich ihrer Winkelausrichtung relativ zur OCT-Austrittsrichtung des Messstrahls aus der OCT-Untersuchungsvorrichtung veränderbar ist und während des Erfassungszeitraums verändert wird, um die Abrasterung durchzuführen, werden bei der erfindungsgemäßen OCT-Untersuchungsvorrichtung die Winkelausrichtung der OCT-Ausgangsrichtung und der OCT-Austrittsrichtung während des gesamten Erfassungszeitraums konstant gehalten. Die erfindungsgemäße OCT-Untersuchungsvorrichtung kann daher auf eine motorisiert oder in anderer Weise bewirkte Verstellung dieser Winkelausrichtung zwischen OCT-Ausgangsrichtung und OCT-Austrittsrichtung verzichten und insbesondere auch auf einen verstellbaren Umlenkspiegel verzichten, was den weiteren Vorteil hat, dass die erfindungsgemäße OCT-Untersuchungsvorrichtung kompakter gestaltet werden kann als vorbekannte OCT-Untersuchungsvorrichtungen. Anstelle der motorischen Verstellungen eines Ablenkspiegels oder eine entsprechend anders aufgebaute, veränderbare Strahlablenkvorrichtung gemäß des Standes der Technik erlaubt die kompakte Ausführung der OCT-Vorrichtung, die Abrasterung der zu untersuchenden Objektoberfläche durch eine Bewegung der OCT-Untersuchungsvorrichtung relativ zu dem zu untersuchenden Objekt durchzuführen. Diese relative Bewegung kann in einer Verschiebung oder Verschwenkung oder einer Kombination hieraus der OCT-Untersuchungsvorrichtung selbst bewirkt werden, ebenso kann aber auch bei räumlich ortsfest gehaltener OCT-Untersuchungsvorrichtung das zu untersuchende Objekt verschoben oder verschwenkt werden, um die Relativbewegung, die für die Abrasterung notwendig ist, zu erzeugen.

Bei der erfindungsgemäßen OCT-Untersuchungsvorrichtung wird der OCT-Messstrahl durch eine Austrittsöffnung hindurch auf das zu untersuchende Objekt gerichtet und die rückgestreute OCT-Strahlung tritt durch eine Eintrittsöffnung wieder in die OCT-Untersuchungsvorrichtung ein. Grundsätzlich ist zu verstehen, dass zwar in bestimmten Anwendungsfällen zwei separate Öffnungen vorgesehen sein können, es ist aber besonders bevorzugt, wenn die Eintrittsöffnung und die Austrittsöffnung durch eine einzige Gehäuseöffnung gebildet werden. Insbesondere können die aus der einzigen Öffnung ausgehende OCT-Strahlung, also der Messstrahl und die rückgestreute OCT-Strahlung koaxial verlaufen, was die OCT-Untersuchungsvorrichtung unempfindlich gegenüber unterschiedlichen Messabständen zwischen der Ein/Austrittsöffnung und der zu untersuchenden Oberfläche macht.

Gemäß einer weiteren bevorzugten Ausführung ist vorgesehen, dass die Austrittsöffnung und die OCT-Strahlungsquelle so angeordnet sind, dass die OCT-Austrittsrichtung und die OCT-Ausgangsrichtung parallel zueinander, insbesondere koaxial verlaufen. Gemäß dieser Ausführungsform kann die aus der OCT-Strahlungsquelle austretende Strahlung unmittelbar in Richtung der Austrittsöffnung ausgerichtet werden und muss nicht mehr umgelenkt werden, um von der OCT-Strahlungsquelle durch die Austrittsöffnung hindurchzutreten. Diese Ausführungsform eignet sich insbesondere für eine schlanke Bauform des Gehäuses, beispielsweise in Form eines langgestreckten, rohrförmigen Gehäuses, welches eine besonders benutzerfreundliche Handhabung der OCT-Untersuchungsvorrichtung ermöglicht.

Gemäß einer weiteren bevorzugten Ausführungsform wird die OCT-Untersuchungsvorrichtung fortgebildet durch eine Beobachtungseinrichtung, umfassend eine Beleuchtungsstrahlungsquelle, die Licht im sichtbaren oder infraroten Beobachtungswellenbereich in einen durch die Austrittsöffnung hindurchtretenden Beleuchtungsstrahlengang aussendet und einen Beobachtungsbildsensor, der für Strahlung im Beobachtungswellenbereich empfindlich ist und der reflektiertes Licht im sichtbaren oder infraroten Beobachtungswellenbereich aus einem durch die Eintrittsöffnung hindurchtretenden Beobachtungsstrahlengang empfängt, wobei der Beobachtungsstrahlengang und die OCT-Austrittsrichtung parallel, insbesondere koaxial zueinander verlaufen. Eine solche Beobachtungseinrichtung ermöglicht es einem Benutzer, das untersuchte Objekt simultan zu der OCT-Bilderfassung durch Abtastung mit einer anderen Untersuchungsmethode, die mit Licht im sichtbaren oder infraroten Bereich die Oberfläche abbildet, zu beobachten. Diese Beobachtungseinrichtung ermöglicht es einerseits, eine simultane Untersuchung mittels zwei unterschiedlicher Abbildungsmethoden durchzuführen, andererseits kann mittels der Beobachtungseinrichtung die Ausrichtung des OCT-Messstrahls auf das zu untersuchende Objekt während der Durchführung der OCT-Untersuchung überwacht werden und der OCT-Messstrahl anhand des von der Beobachtungseinrichtung erfassten Bildes ausgerichtet werden kann. Insbesondere kann die Position des Messstrahls in einer Abbildung, die mittels der Beobachtungseinrichtung erfasst wird, markiert werden. Während der Benutzer ohne das Vorhandensein einer Beobachtungseinrichtung die Abrasterung des zu untersuchenden Objekts ohne eine direkte Positionskontrolle durchführen müsste, kann dies bei Vorhandensein einer Beobachtungseinrichtung gezielt erfolgen und die Steuerungseinheit kann insbesondere dazu ausgebildet sein, um die Ausrichtung des Messstrahls der OCT-Austrittsrichtung und die bisherigen Abrasterungspunkte dieses Messstrahls, also den Verlauf der Abrasterung, in eine Abbildung, die mittels der Beobachtungseinrichtung erstellt wurde, einzublenden.

Die Abrasterung des Untersuchungsobjekts erfolgt erfindungsgemäß durch eine Relativbewegung zwischen der OCT-Untersuchungsvorrichtung und dem Untersuchungsobjekt. Im Gegensatz zu dem Stand der Technik ist es daher nicht erforderlich, dass Untersuchungsobjekt und OCT-Untersuchungsvorrichtung während des Erfassungszeitraums in ihrer Position zueinander konstant bzw. unveränderlich sind und die Abrasterung durch eine interne, veränderliche Ablenkung oder Umlenkung des OCT-Messstrahls innerhalb der OCT-Untersuchungsvorrichtung erfolgt, sondern die Abrasterung und damit Relativbewegung des OCT-Messstrahls erfolgt durch eine Relativbewegung zwischen der OCT-Strahlungsquelle und dem zu untersuchenden Objekt. Diese Relativbewegung kann dabei insbesondere manuell durch einen Benutzer, der die OCT-Untersuchungsvorrichtung händisch führt, erfolgen. Grundsätzlich kann dies in einer vereinfachten Form solcherart durchgeführt werden, dass der Benutzer eine Abrasterungsbewegung manuell und ohne eine visuelle Kontrolle ausführt und sich dabei darauf verlässt, dass er eine solche zeilenweise Abrasterung motorisch zuverlässig ausführen kann. Die manuelle Abrasterung kann aber auch durch eine visuelle Kontrolle unterstützt oder geführt werden, beispielsweise indem dem Benutzer auf einem Bildschirm eine Bewegungsrichtungsanzeige angezeigt wird, welche die Abrasterungsrichtung, die er manuell durchführen sollte, durch einen Pfeil oder dergleichen angezeigt wird oder indem dem Benutzer die bereits mit dem OCT-Messstrahl erfassten Bereiche durch eine entsprechende Wiedergabe des OCT-Untersuchungsbildes angezeigt werden, so dass sich die erfasste Oberfläche des zu untersuchenden Objekts simultan zur Abrasterung entsprechend der Abrasterungsbewegung, die der Benutzer manuell ausführt, aufbaut. Dies ermöglicht es dem Benutzer, den Abrasterungspfad, den er manuell durchführt, anhand des sich aufbauenden OCT-Untersuchungsbildes in Echtzeit oder zeitversetzt zu überprüfen und zu korrigieren und auf diese Weise eine spiralförmige oder zeilenweise oder in anderer geometrischer Form geführte Abrasterung manuell durchzuführen.

Dabei kann die OCT-Untersuchungsvorrichtung weiter fortgebildet werden, indem die elektronische Steuerungseinheit eine Bildbearbeitungseinheit umfasst, die ausgebildet ist, um ein aus der von dem Beobachtungsbildsensor empfangenen, reflektierten Licht gebildetes erstes Bild und ein zeitversetzt zu diesem ersten Bild aus der von dem Beobachtungsbildsensor empfangenen, reflektierten Licht gebildetes, zum ersten Bild benachbartes, zweites Bild zu einem Gesamtbild zusammenzusetzen. Dieser Ausgestaltung der elektronischen Steuerungseinheit ermöglicht es, aus der Rasterungsbewegung, die sich durch die Relativbewegung zwischen der OCT-Untersuchungsvorrichtung bzw. der OCT-Strahlungsquelle und dem zu untersuchenden Objekt ergebenden Aufnahme von mehreren Bildern in einem Zeitversatz zueinander während des Erfassungszeitraums ein Gesamtbild der abgerasterten Oberfläche des untersuchten Objekts zu erstellen. Dieses Zusammensetzen kann durch Aneinanderfügen der einzelnen Bilder erfolgen, wobei sich überlappende Bereiche mit identischen Bildinhalten entsprechend überlappend angeordnet werden bzw. diese Bildinhalte aus nur einem der beiden zusammengefügten Bilder entnommen werden, um eine eindeutige und lückenfreie Erstellung des Gesamtbildes zu erreichen. Die elektronische Steuerungseinheit kann insbesondere dazu ausgebildet sein, um mehrere Bilder zu einem Gesamtbild zusammenzusetzen. Weiterhin kann die elektronische Steuerungseinheit ausgebildet sein, um innerhalb dieser Bilder die mit dem OCT-Messstrahl erfassten Bereiche zu markieren. Weiterhin kann die elektronische Steuerungseinheit ausgebildet sein, um anhand der zusammengesetzten Bilder der Beobachtungseinrichtung auch die mit dem OCT-Messstrahl ermittelten Bildwerte zu einem OCT-Gesamtbild zusammenzusetzen. Dies kann insbesondere solcherart erfolgen, dass anhand der durch Überlappungen festgestellten Positionierung der Bilder der Beobachtungseinrichtung zueinander die Lage der einzelnen OCT-Messpunkte zueinander bestimmt und diese in ihrer Lage zueinander zugeordnet werden.

Noch weiter ist es bevorzugt, wenn die Bildbearbeitungseinheit ausgebildet ist, um einen Überschneidungsbereich des ersten und des zweiten Bildes, in dem ein übereinstimmender Bildabschnitt im ersten und zweiten Bild wiedergegeben ist, zu identifizieren und das erste und zweite Bild solcherart zusammenzusetzen, dass das Gesamtbild aus dem ersten und zweiten Bild mit einer Überlappung des ersten und zweiten Bildes in dem übereinstimmenden Bildabschnitt zusammengesetzt ist. Anhand der so durch die Steuerungseinheit festgestellten übereinstimmenden Bildbereiche kann eine eindeutige Zuordnung der Lage der zwei zusammenzufügenden Bilder in ihrer Relation zueinander durchgeführt werden und daher ein lückenfreies Gesamtbild erstellt werden. Insbesondere ermöglicht die Erkennung von übereinstimmenden Bildinhalten in zwei oder mehr unterschiedlichen Bildern eine eindeutige Zuordnung von OCT-Messpunkten in den jeweiligen Bildern, so dass auf Grundlage einer solchen festgestellten Überlappung auch eine eindeutige Zuordnung der Lage von diesen OCT-Messpunkten erfolgen kann.

Weiterhin kann hierbei vorgesehen sein, dass die Bildbearbeitungseinheit ausgebildet ist, um das erste und/oder das zweite Bild zu entzerren, insbesondere indem das erste und/oder das zweite Bild um eine Bildflächennormale als Schwenkachse verschwenkt wird, und/oder das erste und/oder das zweite Bild skaliert wird, insbesondere in allen Bereichen mit einem übereinstimmenden Skalierungsfaktor skaliert oder mit einem in einer oder in zwei senkrecht zueinander stehenden Raumrichtungen sich verringernden Skalierungsfaktor skaliert wird. Gemäß dieser Fortbildungsform ist die Bildbearbeitungseinheit dazu ausgebildet, um im Zuge der Identifikation übereinstimmender Bildabschnitte eine Entzerrung vorzunehmen, die in einer Verschwenkung oder einer Skalierung oder beidem bestehen kann. Als Skalierung ist hierbei auch eine Skalierung in nur einer Achsrichtung zu verstehen, also eine Skalierung, welche die Seitenlängenverhältnisse des Bildes nicht konstant hält, sondern diese verändert. Durch eine solche Skalierung und Verschwenkung des Bildes können unterschiedliche Aufnahmerichtungen und unterschiedliche Aufnahmemaßstäbe sowie hieraus resultierende zusammengesetzte Verzerrungseffekte ausgeglichen werden und zwei benachbarte Bilder, die durch solche Verzerrungseffekte einen unterschiedlichen Aufnahmewinkel oder -maßstab aufweisen, zusammengefügt werden, ohne dass diese Verzerrungen im Gesamtbild als Fehler auftreten. Insbesondere kann die Steuerungseinheit ausgebildet sein, um einen Abgleich von zwei Bildern in der Prüfung auf übereinstimmende Bildanteile vorzunehmen, welcher solche Verzerrungseffekte berücksichtigt, indem eine Entzerrung der Bilder erfolgt und die vergleichende Analyse der Bilder auf übereinstimmende Bildabschnitte aufgrund der entzerrten Bilder durchgeführt wird.

Noch weiter ist es bevorzugt, wenn die Steuerungseinheit ausgebildet ist, um ein aus der von dem OCT-Strahlungsempfänger empfangenen rückgestreuten OCT-Strahlung erstelltes Messprofil und ein zeitgleich aus dem von dem Beobachtungsbildsensor empfangenen Licht erfasstes Bild zu verarbeiten, und einen Bereich in dem Bild zu markieren, der die Position des Messprofils darstellt. Durch die simultane Verarbeitung der rückgestreuten OCT-Strahlung und des daraus erstellten Messprofils und des von dem Beobachtungsbildsensor empfangenen Lichtes wird eine räumlich eindeutige Zuordnung des OCT-Messprofils in Bezug auf das mit dem Beobachtungsbildsensor erfasste Bild möglich und hierdurch kann eine Aneinanderreihung der einzelnen OCT-Messpunkte anhand der mit dem Beobachtungsbildsensor aufgenommenen Bilder in räumlich korrekter Lage zueinander erfolgen.

Gemäß der Erfindung ist vorgesehen, dass die OCT-Strahlungsquelle und der OCT-Strahlungsempfänger in dem Gehäuse unbeweglich angeordnet sind. Durch eine solche unbewegliche Anordnung wird einerseits eine robuste Ausführung der OCT-Untersuchungsvorrichtung erreicht, weiterhin kann aufgrund dieser räumlich unbeweglichen Anordnung die OCT-Untersuchungsvorrichtung kompakt aufgebaut und in einem schlanken Gehäuse untergebracht werden.

Des weiteren verläuft der OCT-Strahlengang in dem Gehäuse unbeweglich. Durch einen unbeweglichen Verlauf des OCT-Strahlengangs, der sowohl die ausgesendete OCT-Strahlung von der OCT-Strahlungsquelle bis zu der Austrittsöffnung im Gehäuse als auch die empfangene, rückgestreute OCT-Strahlung von der Eintrittsöffnung bis zu dem OCT-Strahlungsempfänger umfasst, wird erfindungsgemäß auf jegliche bewegliche oder steuerbare Ablenkungsmittel für die OCT-Strahlung innerhalb der Untersuchungsvorrichtung verzichtet, was insgesamt einen robusten und schlanken Aufbau der OCT-Untersuchungsvorrichtung ermöglicht. Noch weiter ist es bevorzugt, wenn der OCT-Strahlengang ortsfest in Relation zu dem Beleuchtungsstrahlengang und dem Beobachtungsstrahlengang ist. Durch eine solche ortsfeste, also relativ zueinander unbewegliche Ausführung des OCT-Strahlungsgangs zu dem Beobachtungs- und Beleuchtungsstrahlengang wird erfindungsgemäß eine bereits durch den Aufbau der OCT-Untersuchungsvorrichtung festgelegte klare Zuordnung der Ausrichtung des OCT-Messstrahls und der dadurch definierten Position des OCT-Messpunktes in dem durch die Beobachtungseinrichtung gewonnenen Bildes erzielt.

Noch weiter ist es bevorzugt, die OCT-Untersuchungsvorrichtung fortzubilden durch eine Lageerfassungseinheit, die ausgebildet ist, um die Lage, insbesondere die Position und/oder die Ausrichtung des OCT-Strahlungsempfängers in Bezug auf ein ortsfestes Referenzkoordinatensystem zu ermitteln, und eine Bildbearbeitungseinheit, die mit der Lageerfassungseinheit signaltechnisch gekoppelt und ausgebildet ist, um ein erstes Messprofil aus der von dem OCT-Strahlungsempfänger zu einem ersten Zeitpunkt empfangenen, rückgestreuten OCT-Strahlung zu bestimmen, eine erste Lage des OCT Strahlungsempfängers zum Zeitpunkt des Empfangs der rückgestreuten OCT-Strahlung des ersten Messprofils aus Lagedaten, die von der Lageerfassungeinheit zum ersten Zeitpunkt der Bildbearbeitungseinheit übermittelt werden, zu bestimmen, ein zum ersten Messprofil benachbartes, zweites Messprofil zeitversetzt zu diesem ersten Messprofil aus der von dem OCT-Strahlungsempfänger zu einem zweiten Zeitpunkt empfangenen, rückgestreuten OCT-Strahlung zu bestimmen, eine zweite Lage des OCT Strahlungsempfängers zum Zeitpunkt des Empfangs der rückgestreuten OCT-Strahlung des zweiten Messprofils aus Lagedaten, die von der Lageerfassungeinheit zum zweiten Zeitpunkt der Bildbearbeitungseinheit übermittelt werden, zu bestimmen, die relative Lage des ersten Messprofils zu dem zweiten Messprofil anhand der ersten und zweiten Lage zu bestimmen, und das erste und das zweite Messprofil zu einem Gesamtmessprofil zusammenzusetzen, indem das erste und das zweite Messprofil in der zuvor bestimmten relativen Lage in das Gesamtmessprofil eingetragen werden. Gemäß dieser Ausführungsform umfasst die OCT-Untersuchungsvorrichtung weiterhin eine Lageerfassungseinheit. Diese Lageerfassungseinheit ist ausgestaltet, um eine Lage der OCT-Untersuchungsvorrichtung zu bestimmen. Diese Lagebestimmung kann solcherart durch die Lageerfassungseinheit durchgeführt werden, dass eine relative Änderung der Lage der OCT-Untersuchungsvorrichtung zwischen einem ersten und einem zweiten Zeitpunkt erfolgt, beispielsweise indem eine Beschleunigung der OCT-Untersuchungsvorrichtung oder Beschleunigungen der OCT-Untersuchungsvorrichtung entlang mehrerer Achsen durch einen oder entsprechend mehrere Beschleunigungssensoren, die Bestandteil der Lageerfassungseinheit sind, erfasst wird. Die Lageerfassungseinheit kann auch alternativ oder zusätzlich Sensoren oder einen Sensor umfassen, die/der Ausrichtung der OCT-Untersuchungsvorrichtung in Bezug auf die Schwerkraftrichtung erfassen, um auf diese Weise eine absolute Lageausrichtung der OCT-Untersuchungsvorrichtung zu erfassen. Weiterhin kann die Lageerfassungseinrichtung ein oder mehrere Gyroskope als Sensoren enthalten, um Verschiebungen oder Verschwenkungen entlang mehrerer Achsen zu registrieren.

Die Lageerfassungseinrichtung kann auch solcherart ausgebildet sein, dass sie die Lage der OCT-Untersuchungsvorrichtung in Bezug auf ein von der OCT-Untersuchungsvorrichtung getrenntes und in der Umgebung ortsfest installiertes Referenzsystem bzw. einen entsprechend ortsfest installierten Referenzpunkt erfasst.

Durch die Lageerfassung der OCT-Untersuchungsvorrichtung kann einerseits die Lage, also die Position und Ausrichtung der OCT-Untersuchungsvorrichtung zum Zeitpunkt einer Bilderfassung bestimmt werden und folglich anhand der so bestimmten Lage der OCT-Untersuchungsvorrichtung ein Zusammenfügen mehrerer, in Zeitabständen und unter Veränderung der Lage der OCT-Untersuchungsvorrichtung aufgenommenen Bilder oder OCT-Messprofile durchgeführt werden. Dies kann einerseits anhand von absolut bestimmten Lagen der OCT-Untersuchungsvorrichtung durchgeführt werden, alternativ oder zusätzlich aber auch anhand von relativen Lageänderungen der OCT-Untersuchungsvorrichtung zwischen zwei zeitversetzt aufgenommenen Bilden bzw. OCT-Messpunkten. Es ist zu verstehen, dass diese Zusammenfügung von OCT-Messprofilen zu einem Gesamtmessprofil alleinig anhand der Daten der Lageerfassungseinheit durchgeführt werden kann, die Daten der Lageerfassungseinheit aber auch ergänzend zu einer anderen Vorgehensweise zum Zusammenfügen der einzelnen Messprofile zu einem Gesamtmessprofil herangezogen werden können, beispielsweise indem die Daten der Lageerfassungseinheit ergänzend zu einem Zusammenfügen der Messprofile anhand der mit der Beobachtungseinrichtung aufgenommenen Bilder, der Bestimmung von deren Überlappung und der so definierten Lage der einzelnen OCT-Messprofile zueinander erfolgt.

Ein weiterer Aspekt der Erfindung ist ein Verfahren gemäß der Patentansprüche zur OCT-Bilderfassung eines Gegenstandes mittels optischer Kohärenztomographie, mit den Schritten:
- Aussenden einer OCT-Strahlung mit einer Wellenlänge von 400nm bis 2000nm und einer spektralen Bandbreite, die
   o mindestens einen Bereich von 20nm bis 400nm umfasst, oder
   o eine schmale Bandbreite von weniger als 20nm bis 400nm aufweist, wobei die Strahlungsquelle so durchstimmbar ist, dass die schmale Bandbreite eine breitere Bandbreite von 20nm bis 400nm durch zeitversetzte Aussendung von Wellen in unterschiedlichen Wellenlängen ausbildet,
      aus einer OCT-Strahlungsquelle in einen OCT-Strahlengang, umfassend
   o eine OCT-Ausgangsrichtung der OCT-Strahlung von der OCT-Strahlungsquelle, und
   o eine OCT-Eingangsrichtung einer von einem Bildobjekt rückgestreuten OCT-Strahlung,
- Empfangen der rückgestreuten OCT-Strahlung der OCT-Strahlungsquelle in einem OCT-Strahlungsempfänger,
   wobei ein Gehäuse, das eine Austrittsöffnung und eine Eintrittsöffnung für den OCT-Strahlengang aufweist, die OCT-Strahlungsquelle, den OCT-Strahlungsempfänger aufnimmt,
- Hindurchleiten der OCT-Strahlung durch die Austrittsöffnung in einer OCT-Austrittsrichtung,
- Hindurchleiten der rückgestreuten OCT-Strahlung durch die Eintrittsöffnung in einer OCT-Eintrittsrichtung,
- Ansteuern der OCT-Strahlungsquelle und des OCT-Strahlungsempfänger mittels einer Steuerungseinheit zum Aussenden der OCT-Strahlung mittels der OCT-Strahlungsquelle und zum Empfang der rückgestreuten OCT-Strahlung mittels des OCT-Strahlungsempfänger über einen Erfassungszeitraum, und
- Erfassen mehrerer voneinander beabstandeter Messprofile aus der rückgestreuten OCT-Strahlung während des Erfassungszeitraums, bei dem während des Erfassungszeitraums die OCT-Ausgangsrichtung und die OCT-Austrittsrichtung in ihrer Winkelausrichtung zueinander konstant sind.

Das erfindungsgemäße OCT-Bilderfassungsverfahren zeichnet sich dadurch aus, dass während der Bilderfassung, bei der mehrere OCT-Messpunkte angefahren werden und entsprechend mehrere OCT-Messprofile erfasst werden, keine Veränderung zwischen der Ausgangsrichtung der OCT-Strahlung aus der OCT-Strahlungsquelle und der Austrittsrichtung der OCT-Strahlung aus der OCT-Untersuchungsvorrichtung erfolgt. Wie eingangs erläutert, kann das Verfahren zur OCT-Bilderfassung eines Gegenstands für jegliche OCT-Untersuchungen an Objekten durchgeführt werden, ggf. kann das Verfahren unter Ausschluss von einem Einsatz als Diagnostizierverfahren am menschlichen oder tierischen Körper ausgeführt werden.

Das Verfahren kann dabei fortgebildet werden, indem zwischen der Erfassung des ersten und der Erfassung des zweiten Bildes der Beobachtungsbildsensor relativ zu dem Bildobjekt bewegt, insbesondere verschoben und/oder verschwenkt wird. Die relative Bewegung zwischen dem Beobachtungsbildsensor und dem zu untersuchenden Bildobjekt kann dabei entweder dadurch erfolgen, dass bei ortsfestem Untersuchungsobjekt der Beobachtungsbildsensor bewegt wird, insbesondere die gesamte OCT-Untersuchungsvorrichtung, in welcher der Beobachtungsbildsensor angeordnet ist, bewegt wird, oder das bei feststehendem Beobachtungsbildsensor das Untersuchungsobjekt bewegt wird. Grundsätzlich kann die Relativbewegung auch durch eine Bewegung sowohl des Beobachtungsbildsensors als auch des Untersuchungsobjekts erfolgen.

Gemäß einer weiteren bevorzugten Ausführungsform ist vorgesehen, dass ein erstes Messprofil und ein zweites Messprofil innerhalb des Erfassungszeitraums erfasst wird und das erste und zweite Messprofil in ihrer räumlichen Lage zueinander zugeordnet und zu einem Gesamtmessprofil zusammengefügt werden, insbesondere indem Zeitgleich zur Erfassung des ersten Messprofils ein erstes Bild mittels Aussenden einer Lichtstrahlung im sichtbaren oder infraroten Wellenlängenbereich und Empfangen des von einem Objekt reflektierten Lichtes erfasst wird, zeitgleich zur Erfassung des zweiten Messprofils ein zweites Bild mittels Aussenden einer Lichtstrahlung im sichtbaren oder infraroten Wellenlängenbereich und Empfangen des von dem Objekt reflektierten Lichtes erfasst wird, die Position des ersten Messprofils im ersten Bild markiert wird, die Position des zweiten Messprofils im zweiten Bild markiert wird, das erste und zweite Bild anhand einer Bildanalyse zu einem Gesamtbild zusammengesetzt werden, indem ein übereinstimmender Bildbereich des ersten und des zweiten Bildes überlappend angeordnet wird, und die relative Positionierung des ersten Messprofils zum zweiten Messprofil anhand deren Position in dem Gesamtbild bestimmt und das erste und zweite Messprofil anhand der so bestimmten relativen Positionierung zu einem Gesamtmessprofil zusammengesetzt werden oder indem zeitgleich zur Erfassung des ersten Messprofils eine erste Lage des Gehäuses erfasst wird, zeitgleich zur Erfassung des zweiten Messprofils eine zweite Lage des Gehäuses erfasst wird, die relative Positionierung des ersten Messprofils zum zweiten Messprofil anhand relativen Änderung der ersten Lage zur zweiten Lage des Gehäuses bestimmt und das erste und zweite Messprofil anhand der so bestimmten relativen Positionierung zu einem Gesamtmessprofil zusammengesetzt werden.

Gemäß dieser Ausführungsform werden mehrere Messprofile zu einem Gesamtmessprofil zusammengesetzt und hierdurch eine entsprechende OCT-Untersuchungsdarstellung einer untersuchten Oberfläche des Objekts und eines darunterliegenden, oberflächlichen Volumenbereichs unterhalb dieser untersuchten Oberfläche erzeugt. Das Aneinanderfügen der durch Abrasterung gewonnenen einzelnen Messprofile zu dem Gesamtmessprofil kann hierbei anhand einer Bildanalyse mit der Bestimmung von übereinstimmenden Bildinhalten und einem entsprechenden Zusammenfügen der Einzelbilder zu einem Gesamtbild ("Stiching") oder anhand einer Lagebestimmung der Lage des Gehäuses der OCT-Untersuchungsvorrichtung und entsprechend der Lage des OCT-Messstrahls, der aus diesem Gehäuse austritt, erfolgen oder es kann eine Kombination aus diesen beiden Methoden zur Zusammenfügung der einzelnen Messprofile zu einem Gesamtmessprofil erfolgen.

Die Umsetzung und Vorteile der Erfindung ergeben sich aus der folgenden Figurenbeschreibung, wobei der Schutzumfang der Erfindung allerdings durch die nachfolgenden Patentansprüche definiert ist. In den Figuren sind verschiedene Ausführungsbeispiele der vorliegenden Erfindung dargestellt. Die Figuren, die Beschreibung und die Ansprüche enthalten zahlreiche Merkmale die einzeln oder in Kombination eine Funktion haben. Es ist zu verstehen, dass alle Merkmale zweckmäßigerweise sowohl einzeln zu betrachten und auch zu sinnvollen weiteren Kombinationen zusammengefasst zu verstehen sind.

Es zeigen:

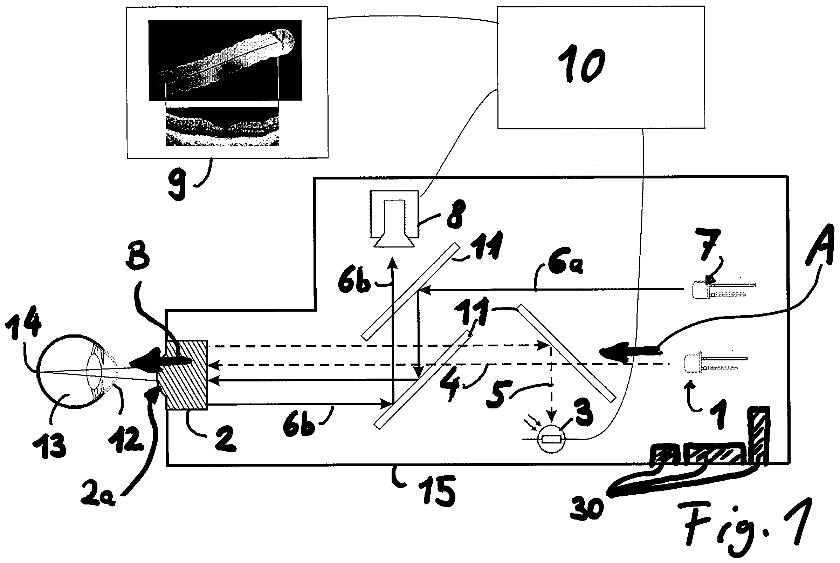

- Fig. 1: einen schematischen Aufbau der erfindungsgemäßen OCT-Vorrichtung. Zwecks besserer Darstellbarkeit ist die OCT-Vorrichtung im Verhältnis größer dargestellt als ein zu vermessendes Auge.

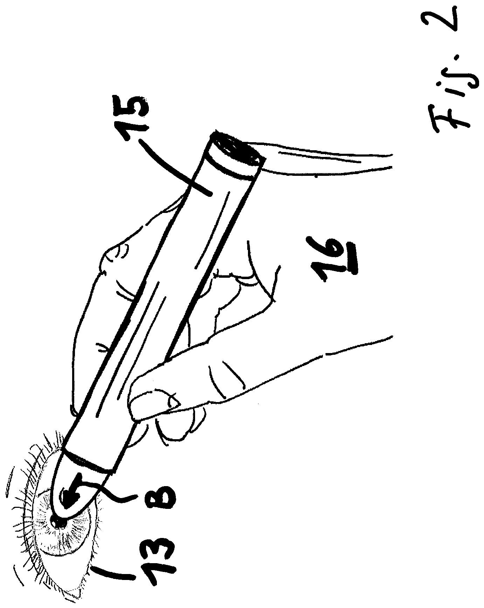

- Fig. 2: ein erstes Ausführungsbeispiel der Erfindung in der Anwendung.

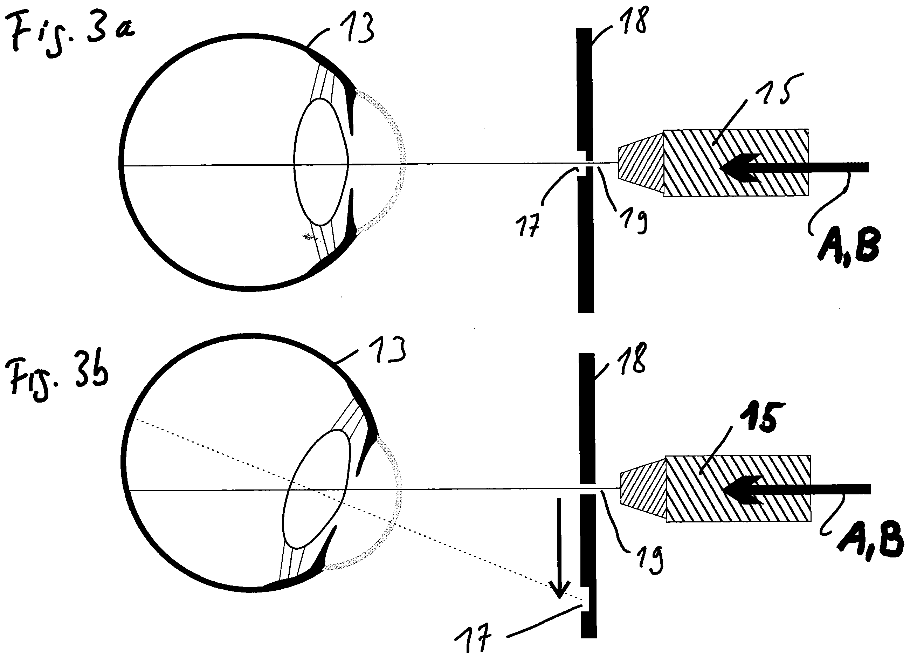

- Fig. 3a u. 3b: ein zweites Ausführungsbeispiel, in welchem der Patient veranlasst wird, mit seinem Auge einem bewegten Fixationslicht zu folgen. Das Fixationslicht kann durch einen Bildschirm im Inneren einer Anordnung erzeugt und bewegt werden.

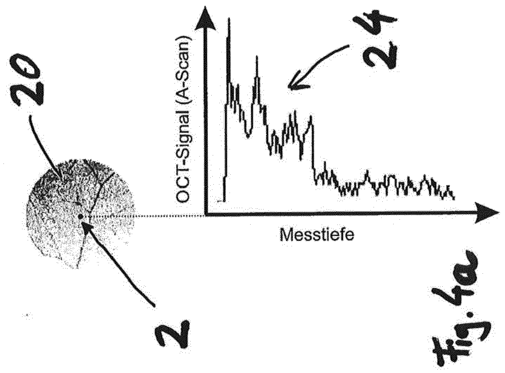

- Fig. 4a: eine beispielhafte Darstellung eines ersten Messergebnisses der erfindungsgemäßen OCT-Vorrichtung als einzelnes Videobild mit zentraler Markierung des OCT-Messpunktes und einem dazugehörigem A-Scan.

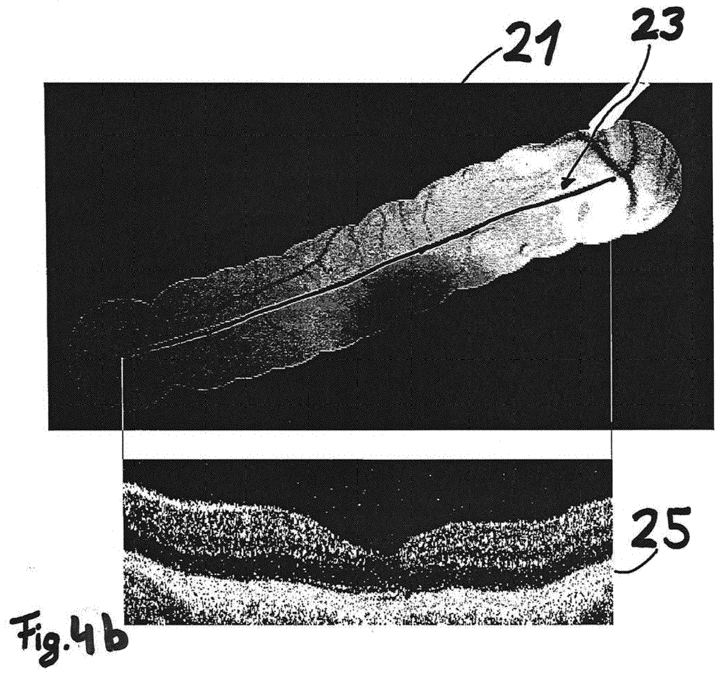

- Fig. 4b: eine beispielhafte Darstellung eines zweiten Messergebnisses der erfindungsgemäßen OCT-Vorrichtung mit aneinander gereihten Videobildern, einer sich ergebenden Linie der OCT-Messpunkte und einem dazugehörigem B-Scan.

Die OCT-Vorrichtung 15 zur Lösung der Aufgabe umfasst eine kurzkohärente Strahlungsquelle (z.B. SLD), (1) die sich durch eine entsprechende Wellenlänge (400 nm - 2.000 nm) und spektrale Bandbreite (20 nm - 400 nm) auszeichnet, um optische Kohärenztomographie (OCT) zu betreiben. Darüber hinaus enthält die OCT-Vorrichtung eine Optik (2), mit der sich der Messstrahl (4) gebündelt auf das Messobjekt richten lässt.

Aus der Strahlungsquelle tritt die OCT-Strahlung in einer OCT-Ausgangsrichtung A aus und trifft auf die Optik. Die OCT-Strahlung durchläuft die Optik und tritt in einer Austrittsrichtung B aus einer Austrittsöffnung 2a aus.

Wahlweise lässt sich statt der breitbandigen Lichtquelle eine schnell durchstimmbare Lichtquelle (sog. Swept-Source) mit geringerer Bandbreite verwenden.

Weiterhin enthält die Anordnung einen OCT-Detektor (3) zur Aufnahme der rückgestreuten OCT-Strahlung (5) des Messstrahls. Im Falle der breitbandigen Lichtquelle kann der OCT-Detektor ein Spektrometer sein, der die rückgestreute OCT-Strahlung spektral zerlegt auf einer linearen Sensorzeile anzeigt (Fourier-Domain-OCT). Im Falle der durchstimmbaren OCT-Strahlungsquelle kann der OCT-Detektor aus einem einfachen, punktförmigen Lichtsensor (Photodiode) bestehen (Swept-Source-OCT). Im sog. Time-Domain-OCT-Modus, in dem die Wegstrecke des Referenzstrahls während der Messung verändert wird, kann der OCT-Detektor ebenfalls aus einem punktförmigen Detektor bestehen.

Kollinear zum OCT-Messstrahl existiert ein Bildgebungsstrahlengang für eine Videoaufnahme. Der Bildgebungsstrahlengang (für sichtbares und infrarotes Licht) wird beispielsweise über teildurchlässige Spiegel (11) eingekoppelt. Er besteht aus Beleuchtungsstrahlengang (6a) und Beobachtungsstrahlengang (6b). Eine Beleuchtung (7) wird beispielsweise durch eine LED im sichtbaren oder infraroten Wellenlängenbereich erzeugt. Das reflektierte Licht (6b) fällt über eine abbildende Optik (2) auf einen lichtempfindlichen Sensor (8), beispielsweise ein CCD-Chip. Die Bildinformation des CCD-Chips wird einerseits direkt auf einem Monitor visualisiert (9), andererseits wird das Bild digital auf einem Datenspeicher in einem Steuerrechner (10) abgespeichert.

Ist beispielsweise die OCT-Vorrichtung auf die Hornhaut (12) des Auges (13) aufgesetzt, ist der Bildgebungsstrahlengang so konzipiert, dass durch ihn ein kleiner Ausschnitt der Netzhaut des Auges (Retina) (14) dargestellt werden kann. Eine Bewegung der Anordnung (15) relativ zum Auge (13), etwa durch Verkippung oder laterale Verschiebung per Hand des Untersuchers (16), beleuchtet ein neues Areal der Netzhaut und stellt dieses Bild entsprechend auf dem Monitor dar und speichert die Information in einem hinreichend schnellem Taktverhältnis auf dem Datenträger im Steuerrechner (10) ab.

Dieser Prozess ist systematisch in etwa vergleichbar mit dem Absuchen einer in der Dunkelheit verborgener Oberfläche mit einem Scheinwerfer. Behält man die beleuchteten Areale in Erinnerung, erhält man ein beliebig großes zusammengesetztes Bild der abgesuchten - abgerasterten - Oberfläche.

Gleichzeitig mit dem freihändigen Abrastern der Netzhautoberfläche durch den Bildgebungsstrahlengang, wird mittels OCT-Messstrahl im Zentrum des beleuchteten Areals bzw. Videoausschnitts ein Tiefenprofil der Netzhaut erzeugt. Der OCT-Messstrahl dringt dabei in die zu vermessende Stelle an der Netzhaut (bzw. einer anderen Probe) ein, ein Teil der OCT-Strahlung wird zurück zum Detektor reflektiert bzw. gestreut. Die rückgestreute OCT-Strahlung wird interferometrisch mit einem Referenzstrahl überlagert. Dadurch entstehen einzelne, axiale Interferogramme. Ein einzelnes Interferogramm (optische Kreuzkorrelation) aus Referenzstrahl und Messstrahl ergibt ein lineares Muster, das die Stärke der lichtreflektierenden Strukturen und deren relative optische Wegstrecke als axiales Tiefenprofil ("A-Scan" oder "amplitude-mode scan") abbildet. Durch Bewegen der Anordnung wird der Messstrahl dann transversal über die Oberfläche der Netzhaut geführt, womit sich ein flächiges Tomogramm ("B.Scan" oder "brightness-mode scan") oder gar ein dreidimensionales Volumen ("c-mode scan") durch Abrasterung aufnehmen lässt.

Alternativ zur Bewegung der OCT-Vorrichtung 15, kann in einer weiteren Ausführung der Patient bei ortsfester OCT-Vorrichtung veranlasst werden, mit seinem Auge einem bewegten Fixationslicht (17) zu folgen. Das Fixationslicht kann durch einen Bildschirm im Inneren einer Anordnung erzeugt und auf dem Bildschirm bewegt werden. Der Messstrahl (4), das rückgestreute Licht (5), sowie der Bildgebungsstrahlengang (6a, 6b) können durch eine zentrale Öffnung (19) auf das Auge (13) und wieder zurück in die Messanordnung (15) gelangen. Durch die Bewegung des Patientenauges entsteht somit ebenfalls eine Abrasterung der Netzhaut.

Da die freihändige Bewegung der beschriebenen Anordnung (bzw. die Bewegung des Auges) und damit das Scannen über die Retina oftmals nicht hinreichend definiert bzw. reproduzierbar erfolgt, ist es vorteilhaft, wenn die dabei entstehenden zweidimensionalen, fotografischen Oberflächenbilder der Netzhaut, sowie die einzelnen axialen Tiefenprofile nachträglich im Steuergerät per entsprechender Software zusammengesetzt werden ("Stitching").

Mit Hilfe des Steuerrechners (10) werden dazu die einzeln aufgenommenen Video-Oberflächenaufnahmen (20) in ein virtuelles Koordinatensystem übertragen und schließlich zu einem Gesamtbild (21) zusammengesetzt. Die Position des OCT-Messstrahls kann in jeder einzelnen Oberflächenaufnahme als Punkt (22) markiert werden. Entsprechend der Bewegung der Anordnung erhält man aus den einzelnen Markierungspunkten eine oder mehrere zusammenhängende Linien (23) auf der Gesamtaufnahme (21). Synchron zu der aufgenommenen Linie kann jeder einzelne Tiefenscan der OCT-Messung (A-Scan) (24) entlang der Linie grafisch aufgetragen werden, um ein entsprechendes flächiges Tomogramm (B-Scan) (25) zu erhalten.

Die Zusammensetzung der Einzelbilder (20) der Oberfläche zu einem Gesamtbild (21) erfolgt derart, dass eine Überlappung zweier benachbarter Bilder in den jeweils übereinstimmenden Bildabschnitten maximiert wird und gegebenenfalls die Einzelbilder für eine kongruente Überlappung entzerrt werden.

Zusätzlich sind mehrere, beispielsweise drei Sensoren (30) in der Anordnung angebracht, die die Bewegung der Anordnung in allen drei Raumrichtungen registrieren und aufzeichnen. Dies kann die Zusammensetzung der Einzelbilder vereinfachen und präzisieren. Derartige Sensoren (30) können beispielsweise Beschleunigungssensoren sein, die translatorische und/oder Rotationsbewegungen registrieren oder die über die Gravitation die Ausrichtung der Anordnung in Bezug auf oben und unten registrieren. Ebenso können als Sensoren (30) Gyroskope zur Registrierung und Verfolgung von Bewegungen der Messanordnung verwendet werden. Mit Hilfe dieser Sensoren kann die Ausrichtung des OCT-Messstrahls und des Abbildungsstrahlengangs zu jedem Messzeitpunkt innerhalb des Erfassungszeitraums der Abrasterung bestimmt werden. Anhand der so bestimmten Ausrichtung können die aufgenommenen Bilder dann zu einem Gesamtbild und die erfassten OCT-Messprofile dann an einem Gesamt-Messprofil zusammengefügt werden.

## Patentansprüche

1. OCT-Untersuchungsvorrichtung (15) zur Erfassung eines Gegenstandes mittels optischer Kohärenztomographie, OCT, umfassend:
- Eine OCT-Strahlungsquelle (1), die ausgebildet ist eine OCT-Strahlung (5) mit einer Wellenlänge von 400nm bis 2000nm und einer spektralen Bandbreite auszusenden, die
o mindestens einen Bereich von 20nm bis 400nm umfasst, oder
o eine schmale Bandbreite von weniger als 20nm bis 400nm aufweist, wobei die Strahlungsquelle (1) so durchstimmbar ist, dass die schmale Bandbreite eine breitere Bandbreite von 20nm bis 400nm durch zeitversetzte Aussendung von Wellen in unterschiedlichen Wellenlängen ausbildet,
- einen OCT-Strahlengang, umfassend
o eine OCT-Ausgangsrichtung (A) der OCT-Strahlung (5) von der OCT-Strahlungsquelle (1), und
o eine OCT-Eingangsrichtung einer von einem Bildobjekt rückgestreuten OCT-Strahlung,
- einen OCT-Strahlungsempfänger (3) für den Empfang der rückgestreuten OCT-Strahlung der OCT-Strahlungsquelle,
- ein Gehäuse, welches die OCT-Strahlungsquelle und den OCT-Strahlungsempfänger aufnimmt,
- eine in dem Gehäuse ausgebildete Austrittsöffnung (2a) der OCT- Strahlung der OCT-Strahlungsquelle,
- eine in dem Gehäuse ausgebildete Eintrittsöffnung der rückgestreuten OCT-Strahlung der OCT-Strahlungsquelle,
- eine OCT-Austrittsrichtung (B) der Strahlung durch die Austrittsöffnung (2a),
- eine OCT-Eintrittsrichtung der rückgestreuten OCT-Strahlung durch die Eintrittsöffnung,
- eine Steuerungseinheit, die signaltechnisch mit der OCT-Strahlungsquelle und dem OCT-Strahlungsempfänger verbunden und ausgebildet ist, um in einem Erfassungszeitraum eine Mehrzahl von voneinander beabstandeten Messprofilen zu erfassen und innerhalb des Erfassungszeitraums die OCT-Strahlungsquelle zum Aussenden der OCT-Strahlung und den OCT-Strahlungsempfänger zum Empfang der rückgestreuten OCT-Strahlung anzusteuern,
wobei die Steuerungseinheit ausgebildet ist, um während des Erfassungszeitraums die OCT-Ausgangsrichtung und die OCT-Austrittsrichtung in ihrer Winkelausrichtung zueinander konstant zu halten, wobei
die OCT-Strahlungsquelle und der OCT-Strahlungsempfänger in dem Gehäuse unbeweglich angeordnet sind und
**dadurch gekennzeichnet, dass**
der OCT-Strahlengang, umfassend die ausgesendete OCT-Strahlung von der OCT-Strahlungsquelle bis zu der Austrittsöffnung im Gehäuse und die empfangene, rückgestreute OCT-Strahlung von der Eintrittsöffnung bis zu dem OCT-Strahlungsempfänger, unbeweglich verläuft indem auf jegliche bewegliche oder steuerbare Ablenkungsmittel für die OCT-Strahlung innerhalb der Untersuchungsvorrichtung verzichtet wird.

2. OCT-Untersuchungsvorrichtung nach Anspruch 1,
**dadurch gekennzeichnet, dass** die Eintrittsöffnung und die Austrittsöffnung (2a) durch eine einzige Gehäuseöffnung gebildet werden.

3. OCT-Untersuchungsvorrichtung nach Anspruch 1 oder 2,
**dadurch gekennzeichnet, dass** die Austrittsöffnung (2a) und die OCT-Strahlungsquelle (1) so angeordnet sind, dass die OCT-Austrittsrichtung (B) und die OCT-Ausgangsrichtung (A) parallel zueinander, insbesondere koaxial verlaufen.

4. OCT-Untersuchungsvorrichtung nach einem der vorhergehenden Ansprüche, **gekennzeichnet durch** eine Beobachtungseinrichtung, umfassend
- eine Beleuchtungsstrahlungsquelle (7), die Licht im sichtbaren oder infraroten Beobachtungswellenbereich in einen **durch** die Austrittsöffnung hindurchtretenden Beleuchtungsstrahlengang (6a) aussendet und
- einen Beobachtungsbildsensor (8), der für Strahlung im Beobachtungswellenbereich empfindlich ist und der reflektiertes Licht im sichtbaren oder infraroten Beobachtungswellenbereich aus einem **durch** die Eintrittsöffnung hindurchtretenden Beobachtungsstrahlengang (6b) empfängt,
wobei der Beobachtungsstrahlengang (6b) und die OCT-Austrittsrichtung (B) parallel, insbesondere koaxial zueinander verlaufen.

5. OCT-Untersuchungsvorrichtung nach Anspruch 4,
**dadurch gekennzeichnet, dass** die elektronische Steuerungseinheit eine Bildbearbeitungseinheit umfasst, die ausgebildet ist, um ein aus der von dem Beobachtungsbildsensor empfangenen, reflektierten Licht gebildetes erstes Bild und ein zeitversetzt zu diesem ersten Bild aus der von dem Beobachtungsbildsensor empfangenen, reflektierten Licht gebildetes, zum ersten Bild benachbartes, zweites Bild zu einem Gesamtbild (21) zusammenzusetzen.

6. OCT-Untersuchungsvorrichtung nach Anspruch 5,
**dadurch gekennzeichnet, dass** die Bildbearbeitungseinheit ausgebildet ist, um einen Überschneidungsbereich des ersten und des zweiten Bildes, in dem ein übereinstimmender Bildabschnitt im ersten und zweiten Bild wiedergegeben ist, zu identifizieren und das erste und zweite Bild solcherart zusammenzusetzen, dass das Gesamtbild aus dem ersten und zweiten Bild mit einer Überlappung des ersten und zweiten Bildes in dem übereinstimmenden Bildabschnitt zusammengesetzt ist.

7. OCT-Untersuchungsvorrichtung nach Anspruch 5 oder 6,
**dadurch gekennzeichnet, dass** die Bildbearbeitungseinheit ausgebildet ist, um das erste und/oder das zweite Bild zu entzerren, insbesondere indem
- das erste und/oder das zweite Bild um eine Bildflächennormale als Schwenkachse verschwenkt wird, und/oder
- das erste und/oder das zweite Bild skaliert wird, insbesondere in allen Bereichen mit einem übereinstimmenden Skalierungsfaktor skaliert oder mit einem in einer oder in zwei senkrecht zueinander stehenden Raumrichtungen sich verringernden Skalierungsfaktor skaliert wird.

8. OCT-Untersuchungsvorrichtung nach Anspruch 5, 6 oder 7,
**dadurch gekennzeichnet, dass** die Steuerungseinheit ausgebildet ist, um ein aus der von dem OCT-Strahlungsempfänger empfangenen rückgestreuten OCT-Strahlung erstelltes Messprofil und ein zeitgleich aus dem von dem Beobachtungsbildsensor empfangenen Licht erfasstes Bild zu verarbeiten, und einen Bereich in dem Bild zu markieren, der die Position des Messprofils darstellt.

9. OCT-Untersuchungsvorrichtung nach einem der vorhergehenden Ansprüche 4-8, **dadurch gekennzeichnet, dass** der OCT-Strahlengang ortsfest in Relation zu dem Beleuchtungsstrahlengang und dem Beobachtungsstrahlengang ist.

10. OCT-Untersuchungsvorrichtung nach einem der vorhergehenden Ansprüche, **gekennzeichnet durch**
- eine Lageerfassungseinheit, die ausgebildet ist, um die Lage, insbesondere die Position und/oder die Ausrichtung des OCT-Strahlungsempfängers in Bezug auf ein ortsfestes Referenzkoordinatensystem zu ermitteln, und
- eine Bildbearbeitungseinheit, die mit der Lageerfassungseinheit signaltechnisch gekoppelt und ausgebildet ist, um
o ein erstes Messprofil aus der von dem OCT-Strahlungsempfänger zu einem ersten Zeitpunkt empfangenen, rückgestreuten OCT-Strahlung zu bestimmen,
o eine erste Lage des OCT Strahlungsempfängers zum Zeitpunkt des Empfangs der rückgestreuten OCT-Strahlung des ersten Messprofils aus Lagedaten, die von der Lageerfassungeinheit zum ersten Zeitpunkt der Bildbearbeitungseinheit übermittelt werden, zu bestimmen,
o ein zum ersten Messprofil benachbartes, zweites Messprofil zeitversetzt zu diesem ersten Messprofil aus der von dem OCT-Strahlungsempfänger zu einem zweiten Zeitpunkt empfangenen, rückgestreuten OCT-Strahlung zu bestimmen,
o eine zweite Lage des OCT Strahlungsempfängers zum Zeitpunkt des Empfangs der rückgestreuten OCT-Strahlung des zweiten Messprofils aus Lagedaten, die von der Lageerfassungeinheit zum zweiten Zeitpunkt der Bildbearbeitungseinheit übermittelt werden, zu bestimmen,
o die relative Lage des ersten Messprofils zu dem zweiten Messprofil anhand der ersten und zweiten Lage zu bestimmen, und
o das erste und das zweite Messprofil zu einem Gesamtmessprofil zusammenzusetzen, indem das erste und das zweite Messprofil in der zuvor bestimmten relativen Lage in das Gesamtmessprofil eingetragen werden.

11. 13. Verfahren zur OCT-Bilderfassung eines Gegenstandes mittels optischer Kohärenztomographie, OCT, mit einer OCT-Untersuchungsvorrichtung mit den Schritten:
- Aussenden einer OCT-Strahlung aus einer OCT-Strahlungsquelle mit einer Wellenlänge von 400nm bis 2000nm und einer spektralen Bandbreite, die
o mindestens einen Bereich von 20nm bis 400nm umfasst, oder
o eine schmale Bandbreite von weniger als 20nm bis 400nm aufweist, wobei die Strahlungsquelle so durchstimmbar ist, dass die schmale Bandbreite eine breitere Bandbreite von 20nm bis 400nm durch zeitversetzte Aussendung von Wellen in unterschiedlichen Wellenlängen ausbildet, in einen OCT-Strahlengang, umfassend
o eine OCT-Ausgangsrichtung der OCT-Strahlung von der OCT-Strahlungsquelle, und
o eine OCT-Eingangsrichtung einer von einem Bildobjekt rückgestreuten OCT-Strahlung,
- Empfangen der rückgestreuten OCT-Strahlung der OCT-Strahlungsquelle in einem OCT-Strahlungsempfänger, wobei ein Gehäuse der OCT-Untersuchungsvorrichtung, das eine Austrittsöffnung und eine Eintrittsöffnung für den OCT-Strahlengang aufweist, die OCT-Strahlungsquelle und den OCT-Strahlungsempfänger aufnimmt,
- Hindurchleiten der OCT-Strahlung durch die Austrittsöffnung in einer OCT-Austrittsrichtung,
- Hindurchleiten der rückgestreuten OCT-Strahlung durch die Eintrittsöffnung in einer OCT-Eintrittsrichtung,
- Ansteuern der OCT-Strahlungsquelle und des OCT-Strahlungsempfänger mittels einer Steuerungseinheit zum Aussenden der OCT-Strahlung mittels der OCT-Strahlungsquelle und zum Empfang der rückgestreuten OCT-Strahlung mittels des OCT-Strahlungsempfänger über einen Erfassungszeitraum,
- Erfassen mehrerer voneinander beabstandeter Messprofile aus der rückgestreuten OCT-Strahlung während des Erfassungszeitraums,
**dadurch gekennzeichnet, dass** während des Erfassungszeitraums die OCT-Ausgangsrichtung und die OCT-Austrittsrichtung in ihrer Winkelausrichtung zueinander konstant sind, indem die OCT-Strahlungsquelle und der OCT-Strahlungsempfänger in dem Gehäuse unbeweglich angeordnet sind und der OCT-Strahlengang, umfassend die ausgesendete OCT-Strahlung von der OCT-Strahlungsquelle bis zu der Austrittsöffnung im Gehäuse und die empfangene, rückgestreute OCT-Strahlung von der Eintrittsöffnung bis zu dem OCT-Strahlungsempfänger, unbeweglich verläuft indem auf jegliche bewegliche oder steuerbare Ablenkungsmittel für die OCT-Strahlung innerhalb der Untersuchungsvorrichtung verzichtet wird.

12. Verfahren zur OCT-Bilderfassung nach Anspruch 11, **dadurch gekennzeichnet, dass** zwischen der Erfassung des ersten und der Erfassung des zweiten Bildes der Beobachtungsbildsensor relativ zu dem Bildobjekt bewegt, insbesondere verschoben und/oder verschwenkt wird.

13. Verfahren zur OCT-Bilderfassung nach Anspruch 11 oder 12, **dadurch gekennzeichnet, dass** ein erstes Messprofil und ein zweites Messprofil innerhalb des Erfassungszeitraums erfasst wird und das erste und zweite Messprofil in ihrer räumlichen Lage zueinander zugeordnet und zu einem Gesamtmessprofil zusammengefügt werden, insbesondere indem:
- Zeitgleich zur Erfassung des ersten Messprofils ein erstes Bild mittels Aussenden einer Lichtstrahlung im sichtbaren oder infraroten Wellenlängenbereich und Empfangen des von einem Objekt reflektierten Lichtes erfasst wird,
- Zeitgleich zur Erfassung des zweiten Messprofils ein zweites Bild mittels Aussenden einer Lichtstrahlung im sichtbaren oder infraroten Wellenlängenbereich und Empfangen des von dem Objekt reflektierten Lichtes erfasst wird,
- Die Position des ersten Messprofils im ersten Bild markiert wird,
- Die Position des zweiten Messprofils im zweiten Bild markiert wird,
- Das erste und zweite Bild anhand einer Bildanalyse zu einem Gesamtbild zusammengesetzt werden, indem ein übereinstimmender Bildbereich des ersten und des zweiten Bildes überlappend angeordnet wird, und
- Die relative Positionierung des ersten Messprofils zum zweiten Messprofil anhand deren Position in dem Gesamtbild bestimmt und das erste und zweite Messprofil anhand der so bestimmten relativen Positionierung zu einem Gesamtmessprofil zusammengesetzt werden,
oder indem
- Zeitgleich zur Erfassung des ersten Messprofils eine erste Lage des Gehäuses erfasst wird,
- Zeitgleich zur Erfassung des zweiten Messprofils eine zweite Lage des Gehäuses erfasst wird,
- Die relative Positionierung des ersten Messprofils zum zweiten Messprofil anhand relativen Änderung der ersten Lage zur zweiten Lage des Gehäuses bestimmt und das erste und zweite Messprofil anhand der so bestimmten relativen Positionierung zu einem Gesamtmessprofil zusammengesetzt werden.

## Claims

1. An OCT examination apparatus (15) for detecting an object by optical coherence tomography, OCT, comprising:
- an OCT radiation source (1) adapted to emit OCT radiation (5) having a wavelength of 400nm to 2000nm and a spectral bandwidth which
∘ comprises at least a range from 20nm to 400nm, or
∘ has a narrow bandwidth of less than 20nm to 400nm, wherein the radiation source (1) is tunable such that the narrow bandwidth forms a wider bandwidth of 20nm to 400nm by time-delayed emission of waves in different wavelengths,
- an OCT beam path comprising
∘ an OCT output direction (A) of the OCT radiation (5) from the OCT radiation source (1), and
∘ an OCT input direction of OCT radiation backscattered from an image object,
- an OCT radiation receiver (3) for receiving the backscattered OCT radiation from the OCT radiation source,
- a housing that accommodates the OCT radiation source and the OCT radiation receiver,
- an exit aperture (2a) for the OCT radiation of the OCT radiation source formed in the housing,
- an entrance aperture formed in the housing for the backscattered OCT radiation of the OCT radiation source,
- an OCT exit direction (B) of the radiation through the exit aperture (2a),
- an OCT entrance direction of the backscattered OCT radiation through the entrance aperture,
- a control unit which is signal-technically connected to the OCT radiation source and the OCT radiation receiver and is designed to detect a plurality of measurement profiles spaced apart from one another in a detection period and to control the OCT radiation source to emit the OCT radiation and the OCT radiation receiver to receive the backscattered OCT radiation within the detection period,
wherein the control unit is adapted to keep the OCT output direction and the OCT exit direction constant in their angular orientation with respect to each other during the detection period, wherein the OCT radiation source and the OCT radiation receiver are immovably arranged in the housing, and **characterised in that** the OCT beam path, comprising the emitted OCT radiation from the OCT radiation source to the exit aperture in the housing and the received, backscattered OCT radiation from the entrance aperture to the OCT radiation receiver, is immobile, **in that** it is refrained from using movable or controllable deflection means for the OCT radiation within the examination device.

2. The OCT examination device according to claim 1,
**characterized in that** the entrance aperture and the exit aperture (2a) are formed by a single housing opening.

3. The OCT examination device according to claim 1 or 2,
**characterized in that** the exit aperture (2a) and the OCT radiation source (1) are arranged in such a way that the OCT exit direction (B) and the OCT output direction (A) extend parallel to one another, in particular coaxially.

4. The OCT examination device according to any of the preceding claims, **characterised by** an observation device comprising
- an illumination radiation source (7) emitting light in the visible or infrared observation waveband into an illumination beam path (6a) passing through the exit aperture, and
- an observation image sensor (8) which is sensitive to radiation in the observation waveband and which receives reflected light in the visible or infrared observation waveband from an observation beam path (6b) passing through the entrance aperture,
wherein the observation beam path (6b) and the OCT exit direction (B) are parallel, in particular coaxial, to each other.

5. The OCT examination device according to claim 4,
**characterised in that** the electronic control unit comprises an image processing unit which is adapted to compose a first image formed from the reflected light received from the observation image sensor and a second image adjacent to the first image formed from the reflected light received from the observation image sensor with a time delay with respect to this first image into an overall image (21).

6. The OCT examination device according to claim 5,
**characterized in that** the image processing unit is configured in order to identify an intersection region of the first and second images, in which a matching image section is reproduced in the first and second images, and to combine the first and second images in such a way that the overall image is composed of the first and second image with an overlap of the first and second images in the matching image section.

7. The OCT examination device according to claim 5 or 6,
**characterised in that** the image processing unit is designed to rectify the first and/or the second image, in particular by
- the first and/or the second image being tilted about an image surface normal as a tilt axis, and/or
- the first and/or the second image being scaled, in particular being scaled in all regions with a matching scaling factor or with a scaling factor decreasing in one or two mutually perpendicular spatial directions.

8. The OCT examination device according to claim 5, 6 or 7,
**characterised in that** the control unit is adapted to process a measurement profile created from the backscattered OCT radiation received by the OCT radiation receiver and an image acquired simultaneously from the light received by the observation image sensor, and to mark an area in the image representing the position of the measurement profile.

9. The OCT examination device according to one of the preceding claims 4-8, **characterized in that** the OCT beam path is stationary in relation to the illumination beam path and the observation beam path.

10. The OCT examination device according to any one of the preceding claims, **characterised by**
- a position detection unit which is designed to determine the position, in particular the position and/or the orientation, of the OCT radiation receiver with respect to a stationary reference coordinate system, and
- an image processing unit which is signal-coupled to the position detection unit and is configured to
∘ determine a first measurement profile from the backscattered OCT radiation received by the OCT radiation receiver at a first time,
∘ determine a first position of the OCT radiation receiver at the time of receiving the backscattered OCT radiation of the first measurement profile from position data transmitted from the position detection unit to the image processing unit at the first time,
∘ to determine a second measurement profile adjacent to the first measurement profile with a time delay relative to this first measurement profile from the backscattered OCT radiation received by the OCT radiation receiver at a second time,
∘ determining a second position of the OCT radiation receiver at the time of receiving the backscattered OCT radiation of the second measurement profile from position data transmitted from the position detection unit to the image processing unit at the second time,
∘ determine the relative position of the first measurement profile to the second measurement profile based on the first and second positions, and
o the first and second measurement profiles are combined to form an overall measurement profile by entering the first and second measurement profiles in the previously determined relative position into the overall measurement profile.

11. A method of OCT imaging of an object by optical coherence tomography, com prising the steps of OCT with an examination device comprising the steps of:. steps:
- Emitting OCT radiation with a wavelength of 400nm to 2000nm and a spectral bandwidth that
∘ comprises at least one range from 20nm to 400nm, or
∘ has a narrow bandwidth of less than 20nm to 400nm, the radiation source being tunable such that the narrow bandwidth forms a wider bandwidth of 20nm to 400nm by time-delayed emission of waves at different wavelengths,
into an OCT beam path comprising
o an OCT output direction of the OCT radiation from the OCT radiation source, and
o an OCT input direction of OCT radiation backscattered from an image object,
- Receiving the backscattered OCT radiation from the OCT radiation source in an OCT radiation receiver,
wherein a housing of the OCT examination device having an exit aperture and an entrance aperture for the OCT beam path accommodates the OCT radiation source and the OCT radiation receiver,
- Passing the OCT radiation through the exit aperture in an OCT exit direction,
- Passing the backscattered OCT radiation through the entrance aperture in an OCT entrance direction,
- Controlling the OCT radiation source and the OCT radiation receiver by means of a control unit for emitting the OCT radiation by means of the OCT radiation source and for receiving the backscattered OCT radiation by means of the OCT radiation receiver over a detection period,
- Acquire multiple spaced measurement profiles from the backscattered OCT radiation during the acquisition period,
**characterised in that** during the detection period the OCT output direction and the OCT exit direction are constant in their angular orientation relative to each other **in that** the OCT radiation source and the OCT radiation receiver are immovably arranged in the housing and the OCT beam path, comprising the emitted OCT radiation from the OCT radiation source to the exit aperture in the housing and the received, backscattered OCT radiation from the entrance aperture to the OCT radiation receiver, runs immovably by dispensing with any movable or controllable deflection means for the OCT radiation within the examination device.

12. The OCT imaging method of claim 11,
**characterised in that** between the acquisition of the first and the acquisition of the second image, the observation image sensor is moved, in particular displaced and/or pivoted, relative to the image object.

13. Method for OCT image acquisition according to claim 11 or 12, **characterised in that** a first measurement profile and a second measurement profile are recorded within the recording period and the first and second measurement profiles are assigned to each other in their spatial position and are combined to form an overall measurement profile, in particular by:
- Simultaneously with the acquisition of the first measurement profile, a first image is acquired by emitting light radiation in the visible or infrared wavelength range and receiving the light reflected from an object,
- Simultaneously with the acquisition of the second measurement profile, a second image is acquired by emitting light radiation in the visible or infrared wavelength range and receiving the light reflected from the object,
- The position of the first measurement profile is marked in the first image,
- The position of the second measurement profile is marked in the second image,
- the first and second images are combined into an overall image based on image analysis by overlapping a matching image area of the first and second images; and
- The relative positioning of the first measurement profile to the second measurement profile is determined based on their position in the overall image and the first and second measurement profiles are combined into an overall measurement profile based on the relative positioning determined in this way,
or by
- A first position of the housing is detected simultaneously with the detection of the first measurement profile,
- A second position of the housing is detected at the same time as the second measurement profile is detected,
- The relative positioning of the first measurement profile to the second measurement profile is determined by means of the relative change of the first position to the second position of the housing and the first and second measurement profiles are combined into an overall measurement profile on the basis of the relative positioning determined in this way.

## Revendications

1. Dispositif d'examen OCT (15) destiné à détecter un objet au moyen d'une tomographie par cohérence optique (OCT), comprenant :
- une source de rayonnement OCT (1), qui est réalisée pour envoyer un rayonnement OCT (5) avec une longueur d'onde de 400 nm à 2000 nm et une largeur de bande spectrale, qui
-- comprend au moins une plage de 20 nm à 400 nm, ou
-- une largeur de bande étroite inférieure à 20 nm à 400 nm,
-- dans lequel la source de rayonnement (1) peut être accordée de telle sorte que la largeur de bande étroite réalise une largeur de bande plus étroite de 20 nm à 400 nm en envoyant de manière décalée dans le temps des ondes dans des longueurs d'onde différentes,
- un chemin optique OCT, comprenant
-- une direction de départ OCT (A) du rayonnement OCT (5) depuis la source de rayonnement OCT (1), et
-- une direction d'entrée OCT d'un rayonnement OCT rétrodiffusé par un objet d'image,
- un récepteur de rayonnement OCT (3) pour la réception du rayonnement OCT rétrodiffusé de la source de rayonnement OCT,
- un boîtier, lequel accueille la source de rayonnement OCT et le récepteur de rayonnement OCT,
- une ouverture de sortie (2a), réalisée dans le boîtier, du rayonnement OCT de la source de rayonnement OCT,
- une ouverture d'entrée, réalisée dans le boîtier, du rayonnement OCT rétrodiffusé de la source de rayonnement OCT,
- une direction de sortie OCT (B) du rayonnement par l'ouverture de sortie (2a) ;
- une direction d'entrée OCT du rayonnement OCT rétrodiffusé par l'ouverture d'entrée,
- une unité de commande, qui est reliée par une technique de signaux à la source de rayonnement OCT et au récepteur de rayonnement OCT et qui est réalisée pour détecter dans une période de détection une multitude de profils de mesure espacés les uns des autres et pour piloter, à l'intérieur de la période de détection, la source de rayonnement OCT pour envoyer le rayonnement OCT et le récepteur de rayonnement OCT pour recevoir le rayonnement OCT rétrodiffusé,
dans lequel
l'unité de commande est réalisée pour maintenir constante l'orientation angulaire de la direction de départ OCT et de la direction de sortie OCT l'une par rapport à l'autre durant la période de détection,
dans lequel
la source de rayonnement OCT et le récepteur de rayonnement OCT sont disposés de manière immobile dans le boîtier, et
**caractérisé en ce que**
le chemin optique OCT, comprenant le rayonnement OCT envoyé depuis la source de rayonnement OCT jusqu'à l'ouverture de sortie dans le boîtier et le rayonnement OCT rétrodiffusé reçu depuis l'ouverture d'entrée jusqu'au récepteur de rayonnement OCT, s'étend de manière immobile **en ce qu'**on renonce à tout moyen de déviation mobile ou pouvant être commandé pour le rayonnement OCT à l'intérieur du dispositif d'examen.

2. Dispositif d'examen OCT selon la revendication 1,
**caractérisé en ce que** l'ouverture d'entrée et l'ouverture de sortie (2a) sont formées par une unique ouverture de boîtier.

3. Dispositif d'examen OCT selon la revendication 1 ou 2,
**caractérisé en ce que** l'ouverture de sortie (2a) et la source de rayonnement OCT (1) sont disposées de telle sorte que la direction de sortie OCT (B) et la direction de départ OCT (A) s'étendent de manière parallèle l'une par rapport à l'autre, en particulier de manière coaxiale.

4. Dispositif d'examen OCT selon l'une quelconque des revendications précédentes,
**caractérisé par** un système d'observation, comprenant
- une source de rayonnement d'éclairage (7), qui envoie de la lumière dans la plage d'ondes d'observation visible ou infrarouge sur un chemin optique d'éclairage (6a) traversant l'ouverture de sortie, et
- un capteur d'image d'observation (8), qui est sensible au rayonnement dans la plage d'ondes d'observation et qui reçoit de la lumière réfléchie dans la plage d'ondes d'observation visible ou infrarouge provenant d'un chemin optique d'observation (6a) traversant l'ouverture d'entrée,
dans lequel le chemin optique d'observation (6b) et la direction de sortie OCT (B) s'étendent de manière parallèle, en particulier de manière mutuellement coaxiale.

5. Dispositif d'examen OCT selon la revendication 4,
**caractérisé en ce que** l'unité de commande électronique comprend une unité de traitement d'image, qui est réalisée pour assembler une première image formée à partir de la lumière réfléchie, reçue par le capteur d'image d'observation et une deuxième image adjacente à la première image, formée de manière décalée dans le temps par rapport à ladite première image à partir de lumière réfléchie reçue du capteur d'image d'observation, pour composer une image globale (21).

6. Dispositif d'examen OCT selon la revendication 5,
**caractérisé en ce que** l'unité de traitement d'image est réalisée pour identifier une plage d'intersection de la première et de la deuxième image, dans laquelle une section d'image concordante est reproduite dans la première et la deuxième image, et pour assembler la première et la deuxième image de telle sorte que l'image globale est composée à partir de la première et de la deuxième image avec un chevauchement de la première et de la deuxième image dans la section d'image concordante.

7. Dispositif d'examen OCT selon la revendication 5 ou 6,
**caractérisé en ce que** l'unité de traitement d'image est réalisée pour corriger la première et/ou la deuxième image, en particulier **en ce que**
- la première et/ou la deuxième image sont pivotées autour d'une normale de surface d'image en tant qu'axe de pivotement, et/ou
- la première et/ou la deuxième image sont mises à l'échelle, en particulier sont mises à l'échelle dans toutes les zones avec un facteur de mise à l'échelle concordant ou avec un facteur de mise à l'échelle diminuant dans une ou dans deux directions spatiales perpendiculaires l'une par rapport à l'autre.

8. Dispositif d'examen OCT selon la revendication 5, 6 ou 7,
**caractérisé en ce que** l'unité de commande est réalisée pour traiter un profil de mesure créé à partir du rayonnement OCT rétrodiffusé reçu par le récepteur de rayonnement OCT et une image détectée simultanément à partir de la lumière reçue par le capteur d'image d'observation, et pour marquer une zone, sur l'image, qui représente la position du profil de mesure.

9. Dispositif d'examen OCT selon l'une quelconque des revendications précédentes 4 - 8, **caractérisé en ce que** le chemin optique OCT est stationnaire par rapport au chemin optique d'éclairage et au chemin optique d'observation.

10. Dispositif d'examen OCT selon l'une quelconque des revendications précédentes,
**caractérisé par**
- une unité de détection de position, qui est réalisée pour déterminer la position, en particulier la position et/ou l'orientation du récepteur de rayonnement OCT par rapport à un système de coordonnées de référence stationnaire, et
- une unité de traitement d'image, qui est couplée par une technique de signaux à l'unité de détection de position et qui est réalisée pour
-- définir un premier profil de mesure à partir du rayonnement OCT rétrodiffusé reçu par le récepteur de rayonnement OCT à un premier moment,
-- définir une première position du récepteur de rayonnement OCT au moment de la réception du rayonnement OCT rétrodiffusé du premier profil de mesure à partir de données de position, qui sont transférées par l'unité de détection de position au premier moment à l'unité de traitement d'image,
-- définir un deuxième profil de mesure adjacent au premier profil de mesure de manière décalée dans le temps par rapport audit premier profil de mesure à partir du rayonnement OCT rétrodiffusé reçu par le récepteur de rayonnement OCT à un deuxième moment,
-- définir une deuxième position du récepteur de rayonnement OCT au moment de la réception du rayonnement OCT rétrodiffusé du deuxième profil de mesure à partir de données de position, qui sont transmises par l'unité de détection de position au deuxième moment à l'unité de traitement d'image,
-- définir la position relative du premier profil de mesure par rapport au deuxième profil de mesure à l'aide de la première et de la deuxième position, et
- assembler le premier et le deuxième profil de mesure en un profil de mesure global en ce que le premier et le deuxième profil de mesure sont entrés dans le profil de mesure global dans la position relative définie au préalable.

11. Procédé de détection d'image OCT d'un objet au moyen d'une tomographie par cohérence optique OCT, avec un dispositif d'examen OCT, avec les étapes :
- d'envoi d'un rayonnement OCT depuis une source de rayonnement OCT avec une longueur d'onde de 400 nm à 2000 nm et une largeur de bande spectrale qui
-- comprend au moins une plage de 20 nm à 400 nm, ou
-- présente une largeur de bande étroite inférieure à 20 nm à 400 nm,
-- dans lequel la source de rayonnement peut être accordée de telle sorte que la largeur de bande étroite réalise une largeur de bande plus étroite de 20 nm à 400 nm par envoi décalé dans le temps d'onde dans des longueurs d'onde différentes,
sur un chemin optique OCT, comprenant
-- une direction de départ OCT du rayonnement OCT de la source de rayonnement OCT, et
-- une direction d'entrée OCT d'un rayonnement OCT rétrodiffusé par un objet d'image, et
- de réception du rayonnement OCT rétrodiffusé de la source de rayonnement OCT dans un récepteur de rayonnement OCT,
dans lequel un boîtier du dispositif d'examen OCT, qui présente une ouverture de sortie et une ouverture d'entrée pour le chemin optique OCT, accueille la source de rayonnement OCT et le récepteur de rayonnement OCT,
- de guidage du rayonnement OCT à travers l'ouverture de sortie dans une direction de sortie OCT,
- de guidage du rayonnement OCT rétrodiffusé à travers l'ouverture d'entrée dans une direction d'entrée OCT,
- de pilotage de la source de rayonnement OCT et du récepteur de rayonnement OCT au moyen d'une unité de commande pour envoyer le rayonnement OCT au moyen de la source de rayonnement OCT et pour recevoir le rayonnement OCT rétrodiffusé au moyen du récepteur de rayonnement OCT sur une durée de détection,
- de détection de plusieurs profils de mesure espacés les uns des autres à partir du rayonnement OCT rétrodiffusé durant la durée de détection,
**caractérisé en ce que** durant la durée de détection, l'orientation angulaire de la direction de départ OCT et de la direction de sortie OCT l'une par rapport à l'autre sont constantes **en ce que** la source de rayonnement OCT et le récepteur de rayonnement OCT sont disposés de manière immobile dans le boîtier et le chemin optique OCT, comprenant le rayonnement OCT envoyé depuis la source de rayonnement OCT jusqu'à l'ouverture de sortie dans le boîtier et le rayonnement OCT rétrodiffusé reçu depuis l'ouverture d'entrée jusqu'au récepteur de rayonnement OCT, s'étend de manière immobile **en ce**
**qu'**on renonce à tout moyen de déviation mobile ou pouvant être commandé pour le rayonnement OCT à l'intérieur du dispositif d'examen.

12. Procédé de détection d'image OCT selon la revendication 11,
**caractérisé en ce que** le capteur d'image d'observation est déplacé, en particulier est décalé et/ou pivoté, par rapport à l'objet d'image entre la détection de la première image et la détection de la deuxième image.

13. Procédé de détection d'image OCT selon la revendication 11 ou 12,
**caractérisé en ce qu'**un premier profil de mesure et un deuxième profil de mesure sont détectés à l'intérieur de la durée de détection, et le premier et le deuxième profil de mesure sont associés l'un à l'autre dans leur position spatiale, et sont assemblés en un profil de mesure global, en particulier **en ce que** :
- dans le même temps que la détection du premier profil de mesure, une première image est détectée au moyen de l'envoi d'un rayonnement lumineux dans la plage de longueurs d'onde visible ou infrarouge et de la réception de la lumière réfléchie par un objet,
- dans le même temps que la détection du deuxième profil de mesure, une deuxième image est détectée au moyen de l'envoi d'un rayonnement lumineux dans la plage de longueurs d'onde visible ou infrarouge et de la réception de la lumière réfléchie par l'objet,
- la position du premier profil de mesure est marquée sur la première image,
- la position du deuxième profil de mesure est marquée sur la deuxième image,
- la première et la deuxième image sont assemblées à l'aide d'une analyse d'image en une image globale, **en ce qu'**une zone d'image concordante de la première et de la deuxième image est disposée de manière à chevaucher, et
- le positionnement relatif du premier profil de mesure par rapport au deuxième profil de mesure est défini à l'aide de sa position sur l'image globale et le premier et le deuxième profil de mesure sont assemblés en un profil de mesure global à l'aide du positionnement relatif ainsi défini
ou **en ce que**
- dans le même temps que la détection du premier profil de mesure, une première position du boîtier est détectée,
- dans le même temps que la détection du deuxième profil de mesure, une deuxième position du boîtier est détectée,
- le positionnement relatif du premier profil de mesure par rapport au deuxième profil de mesure est défini à la l'aide d'une modification relative de la première position par rapport à la deuxième position du boîtier et le premier et le deuxième profil de mesure sont assemblés en un profil global à l'aide du positionnement relatif ainsi défini.
